# EUROPEAN PATENT SPECIFICATION

(11) **EP 2 055 782 B1**
(45) Date of publication and mention of the grant of the patent: **25.08.2010**
(21) Application number: 07021136.2
(22) Date of filing: 30.10.2007
(51) Int. Cl.: C12N 15/82, A01H 5/00

(54) **Method for improving the crop of laticiferous plants**
Verfahren zur Verbesserung des Ertrags von milchsaftführenden Pflanzen
Procédé d'amélioration de la récolte des plantes laticifères

(43) Date of publication of application: 06.05.2009
(73) Proprietor: Fraunhofer-Gesellschaft zur Förderung der angewandten Forschung e.V., 80686 München (DE)
(72) Inventor: Schulze Gronover, Christian, 48143 Münster (DE); Fischer, Rainer, 52156 Monschau (DE)
(74) Representative: Stürken, Joachim

(56) References cited:
- FOUCU, F.: "Taraxacum officinale as an expression system for recombinant proteins: Molecular cloning and functional analysis of the genes encoding the major latex proteins" PHD-THESIS, [Online] 20 June 2007 (2007-06-20), pages 1-110, XP002466641 University RWTH-Aachen, Germany Retrieved from the Internet: URL:http://darwin.bth.rwth-aachen.de/opus3 /frontdoor.php?la=de&source_opus=1769> [retrieved on 2008-01-28]
- YEANG H Y ET AL: "INITIAL PHYSIOLOGICAL CHANGES IN HEVEA LATEX AND LATEX FLOW CHARACTERISTICS ASSOCIATED WITH INTENSIVE TAPPING" JOURNAL OF RUBBER RESEARCH, LEMBAGA GETAH MALAYSIA, KUALA LUMPUR, MY, vol. 30, no. 1, 1982, pages 31-44, XP008087989 ISSN: 1511-1768
- SABY JOHN K ET AL: "Biochemical characterization of sap (latex) of a few Indian mango varieties" PHYTOCHEMISTRY, PERGAMON PRESS, GB, vol. 62, no. 1, January 2003 (2003-01), pages 13-19, XP004397278 ISSN: 0031-9422
- HARIG, L, ET AL.: "Allgemeine Methoden zur genetischen Transformation von Pflanzen und deren Anwendung bei Taraxacum kok-saghyz, Taraxacum officnale, Medicago truncatula und Nigella sativa" STDIENARBEIT, [Online] 2006, pages 1-22, XP002466642 Retrieved from the Internet: URL:www.anja-th.com/de/download-manager/st udienarbeit.pdf> [retrieved on 2008-01-28]
- WITITSUWANNAKUL D ET AL: "Polyphenol oxidases from latex of Hevea brasiliensis: purification and characterization" PHYTOCHEMISTRY, PERGAMON PRESS, GB, vol. 61, no. 2, September 2002 (2002-09), pages 115-121, XP004374206 ISSN: 0031-9422
- VAN BEILEN ET AL: "Establishment of new crops for the production of natural rubber" TRENDS IN BIOTECHNOLOGY, ELSEVIER PUBLICATIONS, CAMBRIDGE, GB, vol. 25, no. 11, 22 October 2007 (2007-10-22), pages 522-529, XP022324138 ISSN: 0167-7799

## Description

The present invention broadly relates to the field of agronomy and is directed to methods specifically designed to improve the crop of laticiferous plants or parts thereof.

Laticiferous plants possess cells known as laticifers that secrete latex, a colorless or milky sap that protects them against pathogens and insect herbivory. Latex comprises a mixture of proteins, carbohydrates, oils and secondary metabolites, but most importantly it contains rubber, which has diverse industrial uses. Although many plant species are known to produce natural rubber in their latex, the rubber tree *Hevea brasiliensis* is currently the only commercial source for natural latex. Malaysia, Thailand and Indonesia are today's major producers of natural rubber accounting for 80% of the world market.

Current rubber supplies are threatened by the increased demand from countries such as China and India, with their rapidly expanding economies, while plantations of *H. brasiliensis* are dwindling due to the tendency to replace them with palm oil trees in order to meet the increasing demand for biofuel. There have been some efforts to increase the amount of latex production, and potential factors controlling the yield and coagulation of *H. brasiliensis* latex have been described (Gidrol, 1994; Silpi, 2006). Another potential threat is South American Leaf Blight (SALB), a fungal disease caused by the ascomycete *Microcyclus* ulei. This inhibits natural rubber production from *H. brasiliensis* on a commercial scale in South and Central America. Since the majority of cultivated *H. brasiliensis* clones in Asia and Africa lack genetic diversity are therefore highly susceptible to this disease, devastating losses are expected if SALB spreads to Asia. To meet these challenges, the identification of alternative sources of natural rubber is an important objective of plant research. Alternative sources are also desirable for individuals with latex allergies.

In the past, research and development programs for rubber production have helped to identify nearly 1800 plant species that contain rubber in their latex. However, only a few of these species are able to produce large amounts of high-molecular-weight rubber. These include the Mexican shrub guayule (*Parthenium argentatum*) and the Russian dandelion (*Taraxacum koksaghyz*). Although *T. koksaghyz* was used intensively in Europe as an alternative source for natural rubber during World War II, the latex extraction procedure is laborious and the latex polymerizes rapidly after wounding.

Accordingly, there is a strong demand for methods that improve the yield or crop of laticiferous plants. In particular, there is a need for methods that enable to crop, collect or harvest latex with a significantly delayed polymerisation time or with an improved latex fluidity, respectively. Furthermore, there is a demand for genetically modified or otherwise treated laticiferous plants in which the latex expelling from laticiferous plant material has a delayed polymerisation time or an improved fluidity, respectively.

The present invention provides methods and laticiferous plants as defined in claims 1 and 7 that help to overcome the problems of the prior art. These methods serve to improve the crop of laticiferous plants or parts thereof. Moreover, the laticiferous plants or parts according to the invention show a delayed polymerisation time or an improved fluidity, respectively, of the latex expelling from respective cells and tissues.

According to one basic principle of the invention, it was found that a major component of the latex protein from laticiferous *Taraxacum species* is represented by a polyphenoloxidase (PPO), and that the level of PPO activity correlates with the rate of latex polymerization. Surprisingly, a comparison of latex flow and the presence of a PPO revealed that latex exhibiting strong PPO activity polymerises rapidly, whereas reduced PPO activity results in a sequential reduction or delay of latex polymerisation. On the basis of these findings, genetically modified (transgenic) dandelion plants were established that showed down-regulation of the PPO activity via RNA interference resulting in a strong delay or significant inhibition in latex polymerisation without affecting rubber biosynthesis.

As disclosed, there is provided a method for improving the crop of latex from laticiferous plants or parts thereof, wherein the improvement consists in using laticiferous plants or parts thereof in which the latex-specific polyphenoloxidase activity, as compared to the respective wild-type or untreated plant or part thereof, is significantly reduced or eliminated.

The person skilled in the art will appreciate that there is a broad range of possibilities to make use of the basic principle that has been established according to the invention. On one hand, the practitioner may generate transgenic laticiferous plants in which the latex-specific PPO activity is significantly reduced or even eliminated. In this regard, the artisan may readily apply different techniques including antisense, knock-out, RNAi and other approaches that aim to reduce or eliminate the PPO activity by affecting or even blocking the transcription and/or translation of the gene or mRNA encoding PPO. Also, (site-directed) mutagenesis and other approaches may be applied. For example, the proteases necessary to cleave the signal or transit peptide can be turned off, blocked or otherwise manipulated, thereby ensuring that no active form of latex-specific PPO is accumulated. Another approach may be to render the latex-specific promoter dysfunctional which may be achieved by blocking or even destroying the relevant section of the respective sequence. Moreover, it may be envisioned to generate transgenic plants or plant material having the ability to express peptides or antibodies that specifically bind to or interact with endogenous latex-specific PPOs such that its activity is significantly reduced.

Thus the laticiferous plants or parts thereof are genetically modified such that the latex-specific polyphenoloxidase activity is significantly reduced or eliminated. The term "significantly reduced" as used herein means that the enzyme's activity in these genetically modified or transgenic plants or parts thereof is lower than in comparable plants or parts of the same origin or taxonomy that have not been subjected to genetic modification and represent the respective wild-type. On a phenotypic level, "significantly reduced" means that the polymerization time of a given amount of expelling latex is significantly delayed. Alternatively, "significantly reduced" or "delayed" means a reduction or a delay of at least 20%.

Preferably, said genetically modified plants or parts thereof contain at least one heterologous nucleotide sequence that is capable of significantly reducing or eliminating the expression of the latex-specific polyphenoloxidase gene.

According to the invention, said at least one heterologous nucleotide sequence encodes at least one RNAi that interferes with the mRNA encoding the latex-specific polyphenoloxidase. As will be appreciated by the skilled person, a suitable RNAi molecule comprises at least 20 to 30 nucleotides and may also be extended to comprise up to 150 or even 300 nucleotides. The design of DNA constructs that encode the RNAi molecules according to the invention can in general be derived from the sequence of SEQ ID No. 1, wherein the RNAi product encoded by said DNA construct or expression cassette is specifically directed against (i) the 5' UTR (position 1 to 1525 of SEQ ID No. 1), (ii) the transit peptide encoding region (position 1526 to 1807 of Seq ID No. 1), (iii) the tyrosinase encoding region (position 1808 to 2896 of SEQ ID No. 1), and/or (iv) the 3' UTR (position 3326 to 3624 of SEQ ID No. 1) of the latex-specific polyphenoloxidase encoding mRNA. According to a preferred embodiment, the RNAi product is specifically directed against at least a portion of the transit peptide encoding region.

As will be explained in more detail hereinbelow, the present specification for the first time discloses and thus provides a latex-specific polyphenoloxidase (PPO) that is represented by SEQ ID Nos. 1 and 2. As will be explained in more detail hereinbelow, the latex-specific polyphenoloxidase according to the invention consists of 599 amino acids and comprises a bipartite transit peptide of 94 amino acids consisting of (i) an envelope transfer peptide (51 amino acids from position 1 to 51 of SEQ ID No. 2) and (ii) a stroma-lumen transfer peptide (43 amino acids from position 52 to 94 of SEQ ID No. 2), a tyrosinase domain (363 amino acids from position 95 to 457 of SEQ ID No. 2), and a C-terminus of unknown function (142 amino acids from position 458 to 599 of SEQ ID NO. 2). The tyrosinase domain has two sets of three histidine residues representing binding regions for two copper ions CuA and CuB (positions 181 to 211 and 333 to 367, respectively, of SEQ ID No. 2).

On the other hand, the skilled artisan guided by the principle according to the invention will appreciate that the aim of significantly reducing or even eliminating the latex-specific PPO activity in laticiferous plants or parts thereof can also be achieved by subjecting the plant material of interest to special conditions which are suitable for significantly reducing or eliminating said activity. For example, the plant material can be placed in an essentially oxygen-free atmosphere.

In addition or alternatively, the laticiferous plants or parts thereof can be harvested in the presence of suitable agents such as chemical substances, inhibitors and/or enzymes that serve for significantly reducing or eliminating the latex-specific PPO activity. With respect to suitable inhibitors, the use of the specific inhibitor 4-hexylresorcinol (Sigma) and/or of structure analogs of catechol is preferred according to the invention. Other preferred inhibitors which act directly on the latex-specific polyphenoloxidase are selected from the group consisting of (i) metal ion chelators, such as azide, cyanide, carbon monoxide, halide ions and tropolone, (ii) aromatic carboxylic acids of the benzoic and cinnamic series, and (iii) mixtures thereof.

In particular, it is preferred to use copper chelators, cinnamic acid, p-coumaric acid, ferulic acid, polyvinylpyrrolidone (PVP and PVPP), salicylhydroxamic acid (SHAM), antioxidants selected from the group consisting of benzoic acid, ascorbic acid, dithiothreitol, and mixtures thereof, and/or mixtures thereof. Furthermore, the use of peptides, proteins or even antibodies that interfere with the latex-specific PPO activity can also be contemplated. For example, the peptide Tentoxin (Sigma) successfully inhibits the processing of the latex-specific PPO .

According to another preferred embodiment of the invention, the laticiferous plants or parts thereof are selected from the group of plant families consisting of Asteraceae, Euphorbiaceae and Moraceae, wherein selection from the group consisting of Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucommia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, and Taraxacum officinale, is particularly preferred.

According to a most preferred embodiment, said laticiferous plants or parts thereof are selected from the group consisting of Hevea brasiliensis, Parthenium argentatum, Sonchus asper, Chrysothamnus nauseosus, Taraxacum koksaghyz, and Taraxacum officinale.

It is clear for a person skilled in the art that the method according to the invention can be carried out both with whole plants or parts thereof, wherein the parts of the plants are preferably selected from the group consisting of cells, tissues, organs, roots, stems, branches, leaves, and mixtures thereof.

According to another aspect, the present invention according to claim 7 provides laticiferous plants or parts thereof being genetically modified such that the latex-specific polyphenoloxidase activity is significantly reduced or eliminated.

The genetically modified laticiferous plant or part thereof contains at least one heterologous nucleotide sequence that is capable of significantly reducing or eliminating the expression of the latex-specific polyphenol-oxidase gene. According to the invention, said at least one heterologous nucleotide sequence encodes at least one RNAi that interferes with the mRNA encoding the latex-specific polyphenoloxidase, wherein said at least one RNAi is specifically directed against (i) the 5' UTR (position 1 to 1525 of SEQ ID No. 1), (ii) the transit peptide encoding region (position 1526 to 1807 of Seq ID No. 1), (iii) the tyrosinase encoding region (position 1808 to 2896 of SEQ ID No. 1), and/or (iv) the 3' UTR (position 3326 to 3624 of SEQ ID No. 1) of the latex-specific polyphenoloxidase encoding mRNA.

Although the invention according to claim 7 broadly provides any genetically modified laticiferous plant with significantly reduced or eliminated latex-specific PPO activity, it is preferred that the laticiferous plant or part thereof is selected from the group consisting of Hevea brasiliensis, Parthenium argentatum, Sonchus asper, Chrysothamnus nauseosus, Taraxacum koksaghyz, and Taraxacum officinale.

In the following, the present invention will be explained in more detail.

Using *T. officinale* as a model organism, a strategy was established to reduce the rate of latex polymerization in transgenic *T. officinale* plants without affecting rubber biosynthesis. This was achieved by using RNAi to downregulate PPO activity. PPOs are found throughout the plant kingdom and they probably play a role in defense against pathogens and herbivores. The wound-inducible expression of PPOs has been reported in apple, tomato, potato and hybrid poplar. In addition, the downregulation of PPO activity by antisense RNA in tomato confers hyper-susceptibility to pathogens (Thipyapong et al., 2004), while PPO overexpression confers enhanced resistance to bacterial disease (Li and Steffens, 2002).

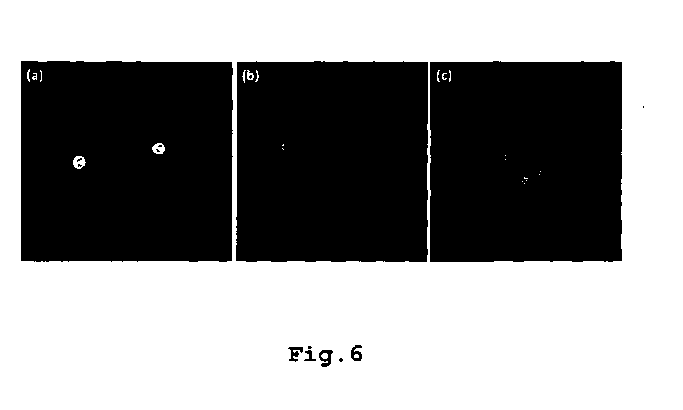

It has also been suggested that PPOs evolved as protection against photochemical oxidation, since all characterized PPOs appear to be localized in the plastids of photosynthetic cells. Interestingly, the immature form of the laticifer- or latex-specific polyphenoloxidase according to the invention TOPPO-1 (indicated by the tissue-specificity of the corresponding promoter) contains a plastid transit peptide even though experiments showed the protein to be laticifer-specific, and laticifers lack conventional plastids. The transit peptide is bipartite, for initial translocation to the chloroplast and subsequent transfer to the thylakoid lumen, indicating a two-step maturation process. Indeed, the TOPPO-1 transit peptide can target reporter proteins into chloroplasts when constitutively expressed in mesophyll cells (Fig. 6). The presence of plastid targeting signals on a laticifer-specific protein strongly suggests that there may be homologous structures in laticifers, a hypothesis supported by electron microscopy data that showed spherical complexes, 3-6 µm in diameter, within *H. brasiliensis* laticifers. These so-called Frey-Wyssling complexes are considered to be specialized plastids due to the presence of a double membrane and the accumulation of carotenes and PPOs. Based on this information and assuming that similar or identical complexes are found in the laticifers of Asteraceae, the following model is proposed: After transcription of the nuclear *toppo-1* gene, the mRNA is translated by ribosomes in the laticifer cytosol, producing the premature (and possibly inactive) form of TOPPO-1 (pre-TOPPO-1). The translocation of pre-TOPPO-1 into the Frey-Wyssling complexes induces sequential processing and the formation of active PPO. In the Frey-Wyssling complexes, PPOs may not have access to substrates that restrict activity in undamaged tissue. However, the pressure drop in wounded laticifers could cause the disruption of Frey-Wyssling complexes followed by the release of active PPO into the cytosol. A similar observation was described for plastids located within the phloem (P plastids).

Several PPOs are known to catalyze the hydroxylation of monophenols to o-diphenols (monophenolase activity) and the oxidation of the o-diphenols to o-quinones (diphenolase activity). These quinones can react with a variety of substances in the laticifer cytosol (e.g. proteins, oligosaccharides) to form a complex brown polymer that is responsible for the rapid wound sealing observed in Asteraceae. The availability of a functional and mature PPO immediately after injury (e.g. by herbivores) would seal the wound rapidly and minimize the risk of secondary infection with facultative pathogens. Clearly PPOs are required for latex polymerization in Asteraceae and a similar role has been suggested for the coagulation of *H. brasiliensis* rubber (Hanower, 1977).

The Russian dandelion *T*. *koksaghyz* has long been considered as a potential alternative source for natural rubber, but the extraction process is expensive and uses chemicals that are harmful to the environment. In contrast thereto and according to the present invention, however, these limitations can now be circumvented through the provision and/or the use of genetically modified *T. koksaghyz* plants with reduced or even no PPO activity, since by carrying out the invention, the latex can be harvested by low-speed centrifugation from mechanically-macerated material and the rubber extracted as described for *H. brasiliensis*. Another advantage of this plant being particularly preferred according to the invention is that it can be cultivated in temperate zones, and the first major field trials have been already initiated in the US (www.deltaplanttechnologies.com). Thus and in accordance with the present invention, the increased demand for natural rubber as well as potential losses in rubber production associated with *H. brasiliensis* (see above) can therefore be accommodated by the provision of optimised alternative rubber plants combined with a good agricultural practise.

### Examples

### 1. Plant material, latex harvest and purification of the aqueous latex interphase

Latex was harvested from greenhouse-cultivated plants by scoring the bark or abscising leaves with a razorblade. Freshly tapped latex was collected in ice-chilled Eppendorf reaction tubes containing 50 mM Tris.HCl, 5mM DTT (pH 6.8). The latex was centrifuged (15,700 rcf, 10 min, 4°C) to separate the aqueous latex interphase from the pellet and (in some species) from an upper viscous layer. In some species the centrifugation step was repeated to remove impurities from the aqueous phase.

### 2. Analysis of T. officinale PPO activity and localization

Latex from *T. officinale* plants was harvested and processed as described above, and the aqueous phase fractionated by 10% SDS-PAGE so that proteins could be visualized by standard Coomassie Blue staining. N-terminal and internal peptide sequences were obtained by blotting proteins onto a PVDF membrane, followed by staining with sulforodamin B (Sigma) and analysis using an ABI Procise 491 Protein Sequencer (Applied Biosystems). Homology searching was carried out using the advanced BLAST program. For the detection of PPO activity, unheated latex proteins were separated by 10% SDS-PAGE, blotted onto nitrocellulose membranes, washed twice with 10 mM Tris.HCl (pH 7.8), 1 mM EDTA, 200 mM NaCl and then incubated in 5 mM L-DOPA, 5 mM 3,4-dihydroxy-L-phenylalanine (L-DOPA; Sigma). When a brown color developed, the reaction was stopped by replacing the L-DOPA solution with water.

For *in situ* PPO assays, semi-thin sections of *T. officinale* petiole were cut with a razor blade and incubated in 5 mM L-DOPA solution. Histochemical localization of GUS activity in transgenic dandelion plants was performed using the substrate 5-bromo-4-chloro-3-indolyl-β-D-glucuronide without an oxidative catalyst, according to established protocols. The stained thin sections were visualized with an Axioskop 40 Zeiss microscope.

### 3. Identification and cloning of toppo-1, and assembly of ptoppo-1, ptoppo-1/GUS and 35S/RNAi constructs

Genomic DNA was extracted from young *T. officinale* leaves using the CTAB method (Doyle, 1990). For the inverse polymerase chain reaction (IPCR), 1 µg of *T. officinale* genomic DNA was digested with different restriction enzymes at 37°C for 2 h. Following phenol:chloroform extraction and ethanol precipitation, the digested DNA was self-ligated at a concentration of 0.3-0.5 µg ml⁻¹ in the presence of 3 U ml⁻¹ T4 DNA ligase (Promega) overnight at 15°C. The ligation mixture was extracted by phenol:chloroform, precipitated with ethanol, and resuspended in water to a concentration of 20 ng µl⁻¹. In order to amplify *toppo*-1 fragments, two degenerate primers were designed on the basis of the peptide amino acid sequences. These were named LZ50 (5'-CAA MCA MCC TMC GTG TCC GGC C-3'; SEQ ID No. 3) and LZ45 (5'-TGG TGI AIG GRG GIC AAC AAT C-3'; SEQ ID No. 4). The IPCR reactions were prepared using 200 ng of recircularized genomic DNA in a final volume of 50 µl containing 50 mM MgCl₂, 0.25 mM of each dNTP, 10 pmol of each primer and 1 unit of *Taq* DNA polymerase. Undigested genomic DNA was used as the control. PCR was initiated by a denaturation step at 94°C for 15 min followed by 36 cycles of denaturation (30 s, 94°C) primer annealing (30 s, 50-60°C) and extension (3 min, 72°C), with a final extension step at 72°C for 10 min. IPCR fragments were cloned in the pGEM-T vector (Promega) and sequenced.

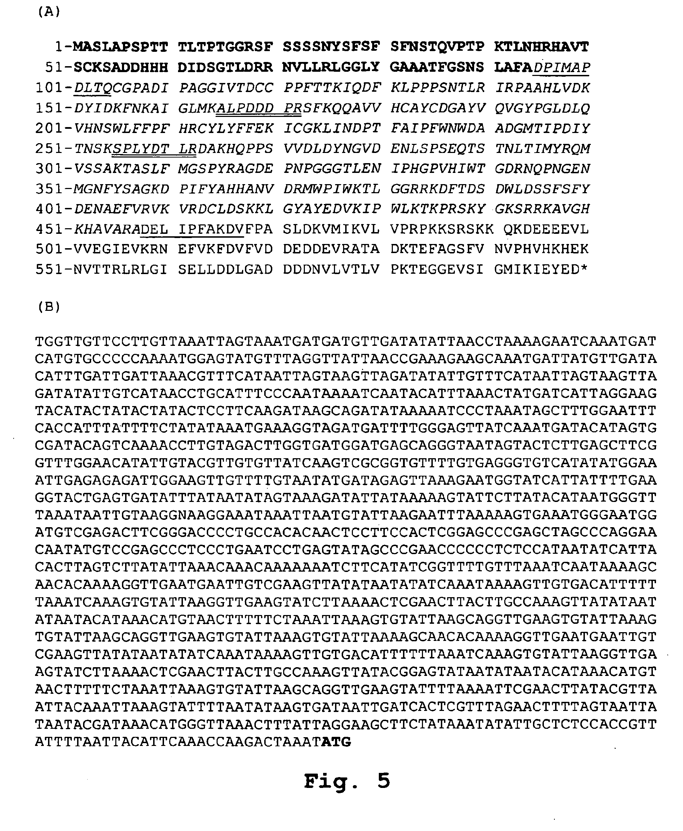

To clone the entire coding region, the *toppo*-1 specific primers LZ60 (5'-GCA AAG GGT ATG AGT TCA TCG G-3'; SEQ ID NO. 5) and LZ62 (5'-AAG ATG TTT TTC CGG CGA GTC T-3'; SEQ ID No. 6), LZ55 (5'-CAT CAT GCG AAT GTC GAT AGA ATG-3'; SEQ ID No. 7) and LZ56 (5'-CCA CCT GCT GGA ATG TCG GC-3'; SEQ ID NO. 8) were used. Total RNA was isolated from the latex of *T. officinale* using Trizol Reagent (Invitrogen) and processed using the OneStep RT-PCR system (Qiagen) and the primers LZ74 (5'-AAA CTC GAG ATA TGG CAT CCC TTG CAC CAT C-3'; SEQ ID No. 9) and LZ73 (5'-CAA TCC TCA TAC TCG ATT TTG ATC-3'; SEQ ID No. 10) following the manufactures instructions. The missing 5' and 3'ends of the *toppo*-1 mRNA were identified using the BD SMART RACE cDNA amplification system (Clontech) and primers 5'-CAG GTG AAC AAT ATT CAT CAG TGG AGC CAC-3' (5' RACE) (SEQ ID No. 11) and 5'-AAA GAT GTT TTT CCG GCG AGT CTT GA-3' (3' RACE) (SEQ ID No. 12) according to the manufactures recommendations. A 1525-bp promoter fragment was isolated using a gene-specific primer (5'- CAG GTG AAC AAT ATT CAT CAG TGG AGC CAC-3')(SEQ ID No. 11) using the Universal GenomeWalker™ Kit (Clontech). This fragment was named ptoppo-1 (Fig. 5b) and represents the sequence immediately upstream of the *toppo-1*translational start codon. For the construction of ptoppo-1/GUS, the *uidA* (*gusA*) gene was placed under the control of ptoppo-1 and ligated into the pTRAk vector to obtain pTRAkptoppo-1/GUS. For the construction of the RNAi construct, a 141-bp PCR fragment was amplified using the RNAi-dicer optimized primers RNAi_for (5'-TTT GGT ACC ATC CAT TGG CCT TTG CAG-3') (SEQ ID No. 13) and RNAi_rev (5'-TTT GCG GCC GCG TGG TGG AAG TTT GAA GTC-3')(SEQ ID No. 14). The PCR fragment was inserted into the *Kpn*I and *Not*I sites of the Gateway vector pENTR4 (Invitrogen) and transferred to the destination plasmid pFGC5941 (http://www.chromDB.org) containing the chalcone synthase intron.

The integrity of all constructs was verified by sequencing. Database analyses were carried out with the Wisconsin Package (Genetics Computer Group). Chloroplast transit and thylakoid lumen targeting signals were analyzed using the ChloroP algorithms (Emanuelsson et al., 1999).

### 4. Plant transformation

*T. officinale* was transformed by *A. tumefaciens*-mediated leaf disc infection. *A. tumefaciens* strain LBA4404, carrying the RNAi construct, was cultured in 100 ml induction broth (5 g l⁻¹ sucrose, 5 g l⁻¹ peptone, 5 g l⁻¹ casein hydrolysate, 1 g l⁻¹ yeast extract, 10 mM MES, 2 mM MgSO₄, pH 5.6) containing 50 mg l⁻¹ kanamycin, 100 mg l⁻¹ rifampicin and 300 mg l⁻¹ streptomycin. The bacteria were cultured at 28°C to stationary phase, then centrifuged, and the pellet was resuspended in coculture medium (4.4 g l⁻¹ Murashige-Skoog salt solution including vitamins, 10 mM MES, 20 g l⁻¹ glucose, pH 5.6), supplemented with 200 µM acetosyringone. Leaf discs (∼1 cm²) were punched from the leaves of 6- to 10-week-old *T. officinale* plants that had been grown under sterile conditions on solid medium (2.2 g l⁻¹ Murashige-Skoog salt solution, 10 g l⁻¹ glucose, 8 g l⁻¹ agar, pH 5.8) and inoculated in the coculture medium with *A*. *tumefaciens* for 30 min. The leaf discs were then placed on filter paper for 14-20 h at 26°C. To induce regeneration, the leaf discs were placed on regeneration medium (4.4 g l⁻¹ Murashige-Skoog salt solution including vitamins, 18 g l⁻¹ glucose, 8.5 g l⁻¹ agar, pH 5.8) supplemented with 1 mg l⁻¹ 6-benzyladenine and 0.2 m gl⁻¹ naphthalenacetic acid for callus and shoot induction. The elongation of shoots was maintained by the addition of 2 mg l⁻¹ zeatin, 20 µg l⁻¹ naphthalenacetic acid and 20 µg l⁻¹ gibberellic acid GA3. Rooting was induced on regeneration medium without hormones, whereas all regeneration media contained 3 mg l⁻¹ phosphinothricin for selection. All chemicals and reagents were purchased from Duchefa.

### 5. Comparison of major latex protein from different laticiferous plants and measurement of latex polymerization

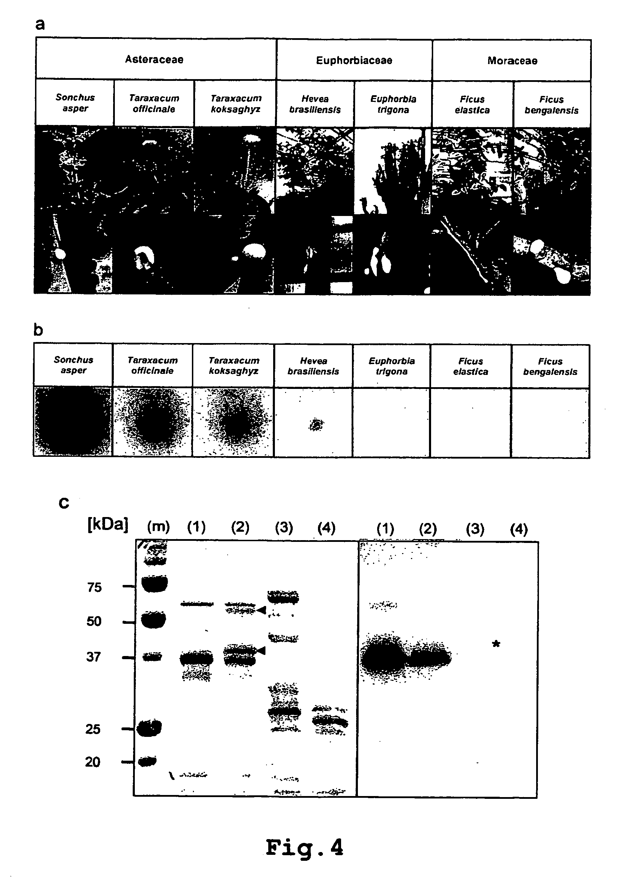

For comparison of the major latex protein of the aqueous phase from different laticiferous plants, 4 µg total protein was applied to polyacrylamide gels and stained with Coomassie Brilliant Blue. To test for enzymatic browning activity, 2 µg total protein from the latex aqueous phase was spotted onto nitrocellulose membranes, dried and soaked in 100 mM phosphate buffer (pH 6) containing 20 mM L-DOPA. In-gel activity studies (Fig. 4c) were performed on 10% SDS gels without heating the latex interphase samples in order to preserve enzymatic activity. After electrophoresis, gels were washed twice for 30 min with 50 mM Tris.HCl (pH 6.8), 1% Triton-X-100, and the wash repeated without Triton-X-100. Enzyme activities were observed by staining gels with 50 mM Tris.HCl (pH 6.8) containing 20 mM L-DOPA.

For the measurement of latex fluidity, petioles from wild type and RNAi plants were dissected and ∼2.5 µl of the latex was drawn ∼20 mm into a glass capillary that was adjusted to a right angled position. The fluidity and polymerization rate of the latex were determined by observing the movement of the latex under gravity within the glass capillary. Latex from *T. koksaghyz* was incubated for 10 min in the presence of 20 µM 4-hexylresorcinol (Sigma) at room temperature and then dropped onto wax foil on an inclined plane. The flow and distance of the latex was compared between untreated latex and latex treated with 4-hexylresorcinol.

### 6. Results

### 1. Analysis of the major latex protein from dandelion

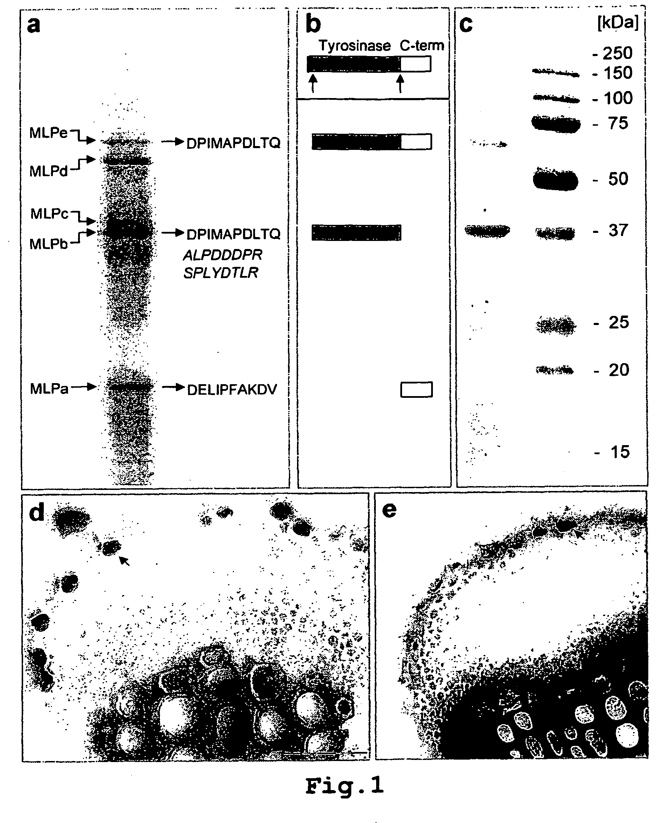

In order to investigate possible correlations between latex polymerization and the presence of specific latex proteins, the major latex proteins (MLPs) from *Taraxacum officinale*, a species whose latex polymerizes rapidly after wounding, was initially analyzed. Stems and leaves were dissected so that latex was expelled, and the latex was then centrifuged and the aqueous phase fractionated by SDS polyacrylamide gel electrophoresis. As shown in Fig. 1a, the aqueous phase contains five dominant protein bands that were designated MLPa (∼16 kDa), MLPb (∼41 kDa), MLPc (∼43 kDa), MLPd (∼52 kDa) and MLPe (∼57 kDa). N-terminal sequencing by Edman degradation identified the N-terminal peptide DELIPFAKDV (SEQ ID No. 15) for MLPa, and DPIMAPDLTQ (SEQ ID No. 16) for both MLPb and MLPe (Fig. 1a). Additionally, two internal peptide sequences were generated from MLPb (Pep1: ALPDDDPR; SEQ ID No. 17; Pep2: SPLYDTLR; SEQ ID No. 18). No sequences were obtained from MLPc and MLPd indicating blockage of the N-terminal residue. Based on this peptide sequence information, either the corresponding full length genomic sequence by inverse PCR or the cDNA sequence by 5' and 3' RACE (rapid amplification of cDNA ends) was cloned using *T. officinale* genomic DNA and cDNA, respectively. Comparison of these sequences revealed the presence of a 1.8-kbp open reading frame, uninterrupted by introns, which encoded a ∼67 kDa protein homologous to polyphenoloxidases. The resulting cDNA clone was thus designated *Taraxacum officinale* polyphenoloxidase 1 (*toppo-1*) and the encoded protein TOPPO-1 (Fig. 1b and Fig. 5; Accession number: EU154993; SEQ ID No. 1). Computational analysis of the protein identified a 10 kDa N-terminal bipartite transit peptide targeting the protein into plastids. The function of the transit peptide was verified by fusion to a reporter protein and subsequent constitutive expression in mesophyll cells (Fig. 1b and Fig. 6). Further analysis showed that MLPa, MLPb and MLPe are generated by proteolytic cleavage of TOPPO-1 with MLPb comprising the tyrosinase domain, MLPa comprising the carboxy-terminal portion of the protein and MLPe comprising both (Fig. 1b). Consequently, only MLPb and MLPe showed PPO activity when incubated with L-DOPA (Fig. 1c). In addition, strong PPO activity was found within the laticifers in thin sections of the petiole of *T. officinale* (Fig. 1d), while transgenic dandelion plants expressing the *gusA* gene under the control of the *toppo-1* promoter (Fig. 5) generated reporter enzyme activity exclusively in laticifers (Fig. 1e). These results show that most of the latex protein is represented by a laticifer-specific PPO enzyme that is probably responsible for the rapid browning and subsequent polymerization of dandelion latex after wounding.

### 2. Downregulation of PPO expression in transgenic dandelion plants and its impact on latex polymerization

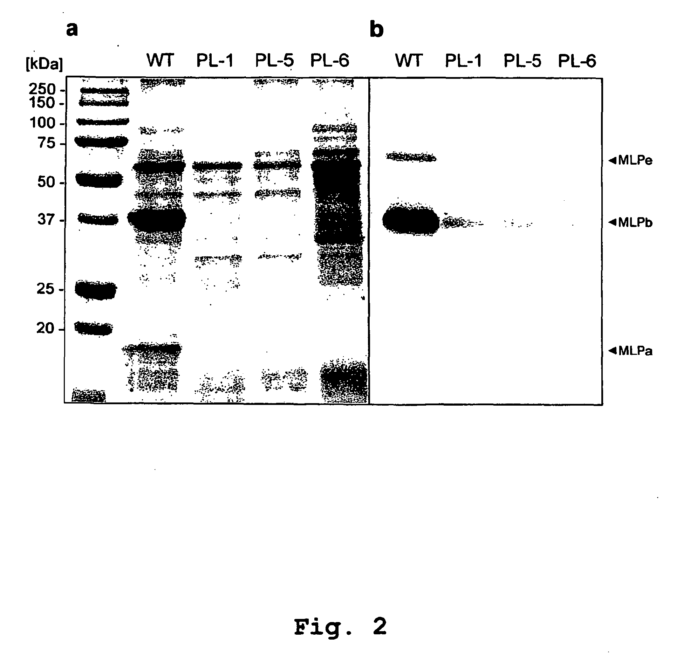

To investigate the relationship between PPO activity and latex polymerization, an RNAi construct was designed to target the tyrosinase domain of *toppo-1* and was expressed under the control of the cauliflower mosaic virus (CaMV) 35S promoter. Twenty-five independent *T. officinale* transgenic lines were generated by *Agrobacterium tumefaciens*-mediated leaf disc transformation. Transgene integration and transcription were confirmed by PCR and RT-PCR, respectively. As expected, there was much variability in the efficiency of *toppo-1* knock-down, ranging from almost complete abolition of PPO activity to near wild-type levels (data not shown). The latex protein composition of transgenic *T. officinale* lines showing the strongest knock-down effects are shown in Fig. 2. The latex of transgenic lines PL-1, PL-5 and PL-6 showed barely detectable levels of MLPa and MLPb. Line PL-6 also featured a unique additional protein band (identified with an asterisk in Fig. 2a) whose origin is unknown. Since MLPb is the most active form of the enzyme in the aqueous latex extracts, a significant reduction in PPO activity was observed in all three transgenic lines. Similar results were obtained for the pre-mature MLPe protein (Fig. 2b). The novel protein band in line PL-6 did not show any PPO activity. None of the transgenic *T. officinale* lines displayed abnormal phenotypes when cultivated in the greenhouse, indicating that the laticifer-specific PPO is unnecessary for normal plant development.

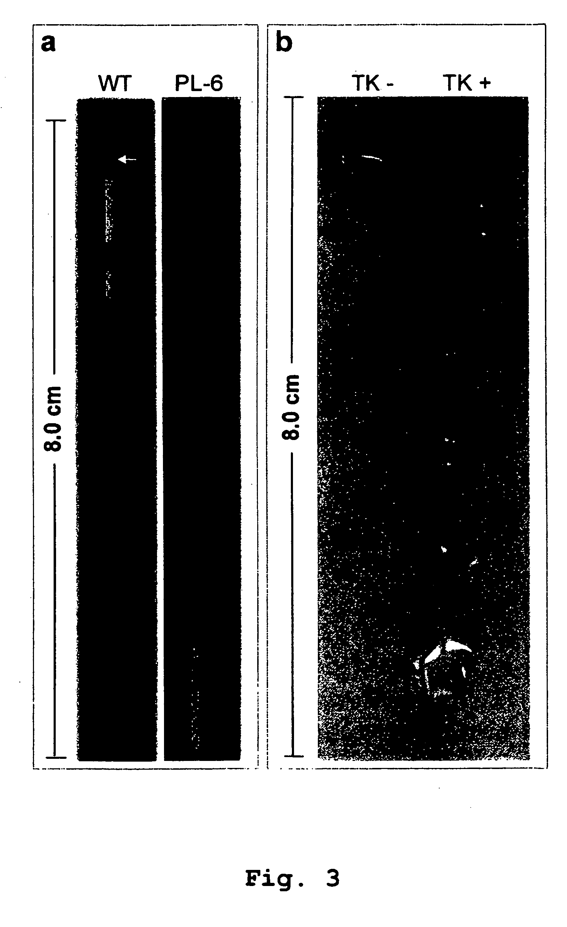

To determine if latex from the transgenic lines polymerized more slowly than wild type latex, the leaves, stems and roots of PL-6 plants were dissected and the flow of latex was evaluated in comparison to wild type plants using a capillary-based test system. Latex from transgenic (PL-6) and wild type plants cultivated under identical conditions was harvested by directly soaking up ∼2.5 µl (∼20 mm) into a narrow glass capillary tube. The gravity-driven flow of latex was determined by measuring the distance between the starting line and the point at which no further latex movement occurred, concomitant with full polymerization (Fig. 3a). The recorded distances were consistent over three measurements and the comparative values recorded for wild type and PL-6 transgenic plants are shown in Table 1. Latex from wild-type plants polymerized soon after soaking up (0.57 ± 0.12 mm; browning at the top of the latex in Fig. 3a) while latex from the transgenic line took almost 30 min to polymerize after loading, which increased the flow distance considerably (7.13 ± 0.35 mm). These results clearly indicated that PPO activity has an important impact on the rate of latex polymerization. However, to exclude the possibility that downregulation of PPO activity might influence rubber biosynthesis, we compared the amount of rubber produced by the transgenic and wild-type plants using a recently described method (Alex, 2003). In three independent experiments, no significant differences in the dry rubber content could be detected (Table 2) indicating that PPO is not necessary for efficient rubber biosynthesis.

Since the aqueous phase of latex from *T. koksaghyz* showed a comparable protein pattern and PPO activity (see Figure 4C), all results obtained for *T. officinale* should also be applicable for *T. koksaghyz* which produces high quantities and qualities of natural rubber. Indeed, initial evidence that the inhibition of PPO activity in *T. koksaghyz* latex can delay polymerization is shown in Figure 3b, where the addition of a PPO-specific inhibitor (4-hexylresorcinol; Martinez-Alvarez, 2005) significantly increases latex flow distances when loaded on an inclined plane. This confirms that PPO activity also influences the polymerization of *T. koksaghyz* latex.

### 3. Correlation between latex polymerization rate and PPO activity in different species

The potential correlation between latex polymerization and PPO activity was investigated in the latex of laticiferous plants from three families: Asteraceae (*Sonchus asper, T. officinale* and *T. koksaghyz*), Euphorbiaceae (*Hevea brasiliensis* and *Euphorbia trigona*) and Moraceae (*Ficus elastica* and *F. bengalensis*). Photographs of greenhouse cultivated plants and of latex expelled from them after injury are shown in Fig. 4a. Polymerization was most rapid in *S. asper* (occurring immediately after dissection) followed by *T. officinale, T. koksaghyz, H. brasiliensis, E. trigona, F. elastica* and finally *F. bengalensis.* The latex from *F. elastica* and *F. bengalensis* had not polymerized fully 30 minutes after leaf dissection.

To determine levels of PPO activity in latex from the same plants, latex aqueous phase samples containing 2 µg of total protein were spotted onto a nitrocellulose membrane and incubated with L-DOPA. As expected (Fig. 4b), the level of PPO activity correlated with the rate of latex polymerization, i.e. species with the faster polymerization rates also had the highest PPO activities. No PPO activity could be detected in the latex from the three species with the slowest polymerization rates (*E. trigona, F. elastica* and *F. bengalensis*).

Subsequently, the major latex proteins were characterized in a selection of laticiferous plants, including those that produce natural rubber and have PPO activity in the latex (*T. officinale, T. koksaghyz* and *H. brasiliensis*) and one species that does not (*F. bengalensis*). As expected from previous experiments (Fig. 3), the major latex protein profiles in *T. koksaghyz* and *T. officinale* were very similar, although two additional major protein bands were observed in *T. koksaghyz* (arrowheads in Fig. 4c). The banding patterns in the other two species were distinct, and while the two *Taraxacum* species showed strong PPO activity associated with the MLPb band, only weak activity was detected in *H. brasiliensis* (indicated by a star) and no activity was detected in *F. bengalensis*. The strongest PPO activity in *H. brasiliensis* was associated with a ∼42 kDa protein which did not correspond to any of the major latex protein bands.

### 7. Description of the Figures and Tables

Figure 1 shows the molecular characterisation of TOPPO-1 from *Taraxacum officinale*. (a) SDS-PAGE gel stained with Coomassie Brillant Blue, showing five dominant bands corresponding to the major latex proteins of *T. offinale* latex (MLPa-e). Peptide sequences obtained by N-terminal sequencing are shown in bold letters. MLPb internal peptide sequences generated for by nanoLC-ESI-MS/MS and MALDI-TOF-MS are shown in italic letters. (b) Schematic representation of TOPPO-1 and its proteolytic products. Cleavage of the pre-TOPPO-1 protein (top) at the sites indicated with arrows produces small amounts of a near full length product (MLPe) lacking the transit peptide (black), together with larger amounts of the tyrosinase domain (MLPb) and C-terminal domain (MLPa, white). (c) PPO activity of the major latex proteins revealed by incubation with 5 mM L-DOPA. As expected, only MLPb and MLPe, which contain the tyrosinase domain, show PPO activity. Molecular weight is shown in kDa. (d) Thin section of a *T. officinale* petiole treated with a 50 mM L-DOPA solution to localize PPO activity, which appears to be laticifer-specific (arrow). (e) Thin section of a transgenic *T. officinale* petiole expressing the *gusA* gene under the control of the *toppo-1* promoter, showing promoter activity specifically in laticifers (arrow).

Figure 2 shows an analysis of PPO abundance and activity in wild-type and RNAi-knockdown *T. officinale* plants. (a) SDS-PAGE showing major latex proteins in wild type (WT) and transgenic plants expressing and RNAi construct targeting the PPO tyrosinase region (PL-1, PL-5, PL-6). Asterisk shows novel and uncharacterized major protein in line PL-6. (b) Activity assay showing proteins with PPO activity. The PPO activity in the RNAi plants is almost completely abolished.

Figure 3 refers to the determination of latex fluidity. (a) Latex from wild type (WT) and transgenic (PL-6) *T. officinale* plants was harvested after wounding by drawing into glass capillaries, which were held in the vertical position to allow gravity flow. After no further movement of the latex, the distance from the end of the capillary to the stopping point was determined (see also Table 1). Visible browning of wild-type latex at the top of the capillary is indicated by an arrow. (b) Influence of the PPO inhibitor 4-hexylresorcinol on *T. koksaghyz* latex polymerization. Latex was harvested from greenhouse-cultivated plants and placed on an inclined glass plate in the absence (TK-) and presence (TK+) of the inhibitor. The entire length of the glass capillary (a) or the declined plane (b) is given in cm.

Figure 4 shows the analysis of Latex and PPO activity in different laticiferous plants. (a) Photographs of S. *asper, T. officinale, T. koksaghyz, H. brasiliensis, E. trigona, F. elastica* and *F. bengalensis*, and of droplets of latex expelled from each plant. Differences in latex fluidity and polymerization are apparent from the size and flow of the droplets. (b) PPO activity visualized by dot blot L-DOPA staining for all the plants listed above. (c) SDS-PAGE and L-DOPA analysis for major latex protein abundance and PPO activity in the rubber-producing plants *T. officinale* (1), *T. koksaghyz* (2), *H. brasiliensis* (3) and *F. elastica* (4). The weak PPO signal for *H. brasiliensis* is indicated with an asterisk. The two additional major proteins in the latex of *T. koksaghyz* are indicated with arrowheads.

Figure 5 shows the amino acid sequence of TOPPO-1 (A) (bold = signal peptide; italic = tyrosinase domain; regular diction = C-terminus)(N-terminal peptide sequences generated by Edman degradation are single underlined; those of internal peptides are double underlined), and the promoter sequence of *toppo-1* (B). The translational start codon is indicated in bold.

Figure 6 shows functional analysis of the signal peptide *in planta*. (a) Expression of a CAMV 35S - signal peptide/DsRed construct in leaves of *Nicotiana tabacum* by agroinfiltration. DsRed fluorescence was visualized with an LCD lamp (Leica KL1500). Confocal laser scanning microscopy (CLSM) analysis of *N. tabacum* (b) and *T. officinale* (c) mesophyll cells expressing the signal peptide/DsRed translational fusion protein identifies DsRed fluorescence in chloroplasts 11 days post inoculation.

Table 1 displays the polymerization of latex from wild type *T. officinale* plants and transgenic line PL-6 (lacking PPO activity) established by gravity flow along a glass capillary. Latex from each plant (∼2.5 µl) was drawn into a capillary, which was then placed vertically to allow the latex to flow under gravity. The distance [cm] of the latices was measured once the flow of latex had stopped.

Table 2 compares the dry rubber content of latex from wild type *T. officinale* plants and transgenic line PL-6 (lacking PPO activity). The dry rubber content of latex was determined as described using 100 µl of latex supplemented with 13.3 µl of 15% potassium oleate and titrated to pH 8.0 with sulfuric acid. The amount of sulfuric acid required until the rubber coagulates gives the dry rubber content by the equation: DRC%=23.59*V (H₂SO₄)[ml] -6,156 (Prokofjew, 1947).

**Table 1**

| **Experiment No.** | **Propagation [cm]** | |
|---|---|---|
| | **WT** | **PL-6** |
| 1 | 0.7 | 7.5 |
| 2 | 0.5 | 7.1 |
| 3 | 0.5 | 6.8 |
| **mean ± deviation** | **0.57± 0.12** | **7.13 ± 0.35** |

**Table 2**

| **Experiment No.** | **DRC [%]** | |
|---|---|---|
| | **WT** | **PL-6** |
| 1 | 3.28 | 3.28 |
| 2 | 2.10 | 3.28 |
| 3 | 2.10 | 2.10 |
| **mean ± deviation** | **2.49 ± 0.68** | **2.89 ± 0.68** |

### References

1. Gidrol, X., Chrestin, H., Tan, H.L. & Kush, A. Hevein, a lectin-like protein from Hevea brasiliensis (rubber tree) is involved in the coagulation of latex. J. Biol. Chem. 25, 9278-9283 (1994).
2. Silpi, U. et al. Effect of tapping activity on the dynamics of radial growth of Hevea brasiliensis trees. Tree Physiol. 26, 1579-1587 (2006).
3. Alex, R., Premalatha, C.K., Nair, R.B. & Kuriakose, B. Measurement of dry rubber content of fresh natural rubber latex by a titration method. J. Rubber Res. 6, 221-230 (2003).
4. Prokofjew, A.A. Veröff. Akad. Wiss. UDSSR (N.S.) 56, 209-212 (1947).
5. Martínez-Álvarez, O., Gómez-Guillén, M.C. & Montero, P. Role of sulfites and 4-hexylresorcinol in microbial growth and melanosis prevention of deepwater pink shrimp (Parapenaeus longirostris) using a controlled atmosphere. J. Food Prot. 68, 98-104 (2005).
6. Thipyapong, P., Hunt, M.D. & Steffens, J.C. Antisense downregulation of polyphenol oxidase results in enhanced disease susceptibility. Planta 220, 105-117 (2004).
7. Li ,L. & Steffens, J.C. Overexpression of polyphenol oxidase in transgenic tomato plants results in enhanced bacterial disease resistance. Planta 215, 239-247 (2002).
8. Hanower, P. & Brzozowska, J. Les phenol-oxydases et la coagulation. In: Colloque sur la physiologie du latex d'Hevea brasiliensis (Institut francais de rechereche scientifique pour le développeement et coopération, Ostrom, 1977, 25-34).
9. Doyle, J.J. & Doyle J.L. Isolation of plant DNA from fresh tissue. Focus 12, 13-15 (1990).
10. Emanuelsson, O., Nielsen, H. & von Heijne, G. ChloroP, a neural network-based method for predicting chloroplast transit peptides and their cleavage sites. Protein Sci. 8, 978-984 (1999).

## Claims

1. Method for improving the crop of latex from laticiferous plants or parts thereof by using genetically modified laticiferous plants or parts thereof containing at least one heterologous nucleotide sequence encoding at least one RNAi molecule that interferes with the latex-specific polyphenoloxidase (PPO) activity, resulting in delayed polymerization time or improved fluidity, respectively, of the latex expelling from said plants or parts thereof as compared to the respective wild-type plants or parts thereof, wherein said at least one RNAi is specifically directed against (i) the 5' or 3' UTR of the latex-specific polyphenoloxidase mRNA transcript, (ii) the mRNA nucleotide sequence that encodes the tyrosinase domain, and/or (iii) the mRNA nucleotide sequence that encodes the transit peptide of the latex-specific polyphenoloxidase.

2. Method according to claim 1, wherein the latex-specific polyphenoloxidase is represented by SEQ ID No. 2.

3. Method according to claim 1 or 2, wherein said laticiferous plants or parts thereof are selected from the group of plant families consisting of Asteraceae, Euphorbiaceae and Moraceae.

4. Method according to claim 3, wherein said laticiferous plants or parts thereof are selected from the group consisting of Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucommia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, and Taraxacum officinale.

5. Method according to claim 3 or 4, wherein said laticiferous plants or parts thereof are selected from the group consisting of Hevea brasiliensis, Parthenium argentatum, Sonchus asper, Chrysothamnus nauseosus, Taraxacum koksaghyz, and Taraxacum officinale.

6. Method according to any of the preceding claims, wherein the parts of the plants are selected from the group consisting of cells, tissues, organs, roots, stems, branches, leaves, and mixtures thereof.

7. Laticiferous plant or part thereof being genetically modified to contain at least one heterologous nucleotide sequence encoding at least one RNAi molecule that interferes with the latex-specific polyphenoloxidase (PPO) activity, resulting in delayed polymerization time or improved fluidity, respectively, of the latex expelling from said plant or part thereof as compared to the respective wild-type plant or part thereof, wherein said at least one RNAi is specifically directed against (i) the 5' or 3' UTR of the latex-specific polyphenoloxidase mRNA transcript, (ii) the mRNA nucleotide sequence that encodes the tyrosinase domain, and/or (iii) the mRNA nucleotide sequence that encodes the transit peptide of the latex-specific polyphenoloxidase.

8. Laticiferous plant or part thereof according to claim 7, wherein the latex-specific polyphenoloxidase is represented by SEQ ID No. 2.

9. Laticiferous plant or part thereof according to claim 7 or 8, selected from the group of plant families consisting of Asteraceae, Euphorbiaceae and Moraceae.

10. Laticiferous plant or part thereof according to claim 9, selected from the group consisting of Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucommia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, and Taraxacum officinale.

11. Laticiferous plant or part thereof according to claim 9 or 10, selected from the group consisting of Hevea brasiliensis, Parthenium argentatum, Sonchus asper, Chrysothamnus nauseosus, Taraxacum koksaghyz, and Taraxacum officinale.

12. Laticiferous plant or part thereof according to any of claims 7 to 11, wherein the part of the plant is selected from the group consisting of cells, tissues, organs, roots, stems, branches, leaves, and mixtures thereof.

## Patentansprüche

1. Verfahren zur Verbesserung der Ernte von Latex aus Milchsaft führenden Pflanzen oder Teilen davon unter Verwendung von genetisch modifizierten Milchsaft führenden Pflanzen oder Teilen davon, die mindestens eine heterologe Nukleotidsequenz enthalten, welche für mindestens ein RNAi Molekül kodiert, das mit der Aktivität der Latex-spezifischen Polyphenoloxidase (PPO) interferiert, woraus eine verzögerte Polymerisationszeit oder verbesserte Fluidität des aus den Pflanzen oder Teilen davon austretenden Latex im Vergleich zu entsprechenden Wildtyp-Planzen oder Teilen davon resultiert, wobei die mindestens eine RNAi spezifisch gegen (i) die 5'- oder 3'-UTR des Latex-spezifischen Polyphenoloxidase mRNA Transkripts, (ii) die für die Tyrosinase-Domäne kodierende mRNA Nukleotidsequenz, und/oder (iii) die für das Transitpeptid der Latex-spezifischen Polyphenoloxidase kodierende mRNA Nukleotidsequenz gerichtet ist.

2. Verfahren nach Anspruch 1, wobei die Latex-spezifische Polyphenoloxidase durch SEQ ID Nr. 2 repräsentiert ist.

3. Verfahren nach Anspruch 1 oder 2, wobei die Milchsaft führenden Pflanzen oder Teile davon aus der Gruppe von Pflanzenfamilien bestehend aus Asteraceae, Euphorbiaceae und Moraceae ausgewählt werden.

4. Verfahren nach Anspruch 3, wobei die Milchsaft führenden Pflanzen oder Teile davon ausgewählt werden aus der Gruppe bestehend aus Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucomnia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, und Taraxacum officinale.

5. Verfahren nach Anspruch 3 oder 4, wobei die Milchsaft führenden Pflanzen oder Teile davon ausgewählt werden aus der Gruppe bestehend aus Hevea brasiliensis, Parthenium argentatum, Sonchus asper, Chrysothamnus nauseosus, Taraxacum koksaghyz, und Taraxacum officinale.

6. Verfahren nach einem der vorhergehenden Ansprüche, wobei die Teile der Pflanzen ausgewählt werden aus der Gruppe bestehend aus Zellen, Geweben, Organen, Wurzeln, Stämmchen, Zweigen, Blättern, und Mischungen davon.

7. Milchsaft führende Pflanze oder Teil davon, die bzw. das genetisch modifiziert ist, um mindestens eine heterologe Nukleotidsequenz zu enthalten, welche für mindestens ein RNAi Molekül kodiert, das mit der Aktivität der Latex-spezifischen Polyphenoloxidase (PPO) interferiert, woraus eine verzögerte Polymerisationszeit oder verbesserte Fluidität des aus der Pflanze oder dem Teil davon austretenden Latex im Vergleich zu der entsprechenden Wildtyp-Planze oder dem Teil davon resultiert, wobei die mindestens eine RNAi spezifisch gegen (i) die 5'- oder 3'-UTR des Latex-spezifischen Polyphenoloxidase mRNA Transkripts, (ii) die für die Tyrosinase-Domäne kodierende mRNA Nukleotidsequenz, und/oder (iii) die für das Transitpeptid der Latex-spezifischen Polyphenoloxidase kodierende mRNA Nukleotidsequenz gerichtet ist.

8. Milchsaft führende Pflanze oder Teil davon nach Anspruch 7, wobei die Latex-spezifische Polyphenoloxidase durch SEQ ID Nr. 2 repräsentiert ist.

9. Milchsaft führende Pflanze oder Teil davon nach Anspruch 7 oder 8, ausgewählt aus der Gruppe von Pflanzenfamilien bestehend aus Asteraceae, Euphorbiaceae und Moraceae.

10. Milchsaft führende Pflanze oder Teil davon nach Anspruch 9, ausgewählt aus der Gruppe bestehend aus Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucomnia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, und Taraxacum officinale.

11. Milchsaft führende Pflanze oder Teil davon nach Anspruch 9 oder 10, ausgewählt aus der Gruppe bestehend aus Hevea brasiliensis, Parthenium argentatum, Sonchus asper, Chrysothamnus nauseosus, Taraxacum koksaghyz, und Taraxacum officinale.

12. Milchsaft führende Pflanze oder Teil davon nach einem der Ansprüche 7 bis 11, wobei das Teil der Pflanze ausgewählt ist aus der Gruppe bestehend aus Zellen, Geweben, Organen, Wurzeln, Stämmchen, Zweigen, Blättern, und Mischungen davon.

## Revendications

1. Méthode pour améliorer la récolte du latex des plantes laticifères ou des parties de celles-ci en utilisant des plantes laticifères modifiées génétiquement ou des parties de celles-ci contenant au moins une séquence nucléotidique hétérologues codant pour au moins une molécule d'ARNi qui interfère avec l'activité polyphenoloxidase (PPO) spécifique du latex, résultant dans un temps de polymérisation retardé ou une amélioration de la fluidité, respectivement, du latex sécrété par les dites plantes ou parties de celles-ci comparé aux plantes sauvages respectives ou parties de celles-ci, dans laquelle le dit ARNi, au moins un, est spécifiquement dirigé contre (i) l'UTR 5' ou 3' du transcrit ARNm de la polyphenoloxidase spécific au latex, (ii) la séquence nucléotidique de l'ARNm qui code pour le domaine tyrosinase, et/ou (iii) la séquence nucléotidique de l'ARNm qui code pour le peptide de transition de la polyphenoloxidase spécifique au latex.

2. Méthode selon la revendication 1 dans laquelle la polyphenoloxidase spécifique du latex est représentée par la SEQ ID No. 2.

3. Méthode selon la revendication 1 ou 2 dans laquelle les dites plantes laticifères ou parties de celles-ci sont sélectionnées dans le groupe de familles de plantes comprenant les astéracées, les euphorbiacées, et les moracées.

4. Méthode selon la revendication 3 dans laquelle les dites plantes laticifères ou parties de celles-ci sont sélectionnées dans le groupe comprenant Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucomnia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, et Taraxacum officinale.

5. Méthode selon la revendication 3 ou 4 dans laquelle les dites plantes laticifères ou parties de celles-ci sont sélectionnées dans le groupe comprenant Hevea brasiliensis, Parthenium argentatum, Sonchus asper, Chrysothamnus nauseosus, Taraxacum koksaghyz, et Taraxacum officinale.

6. Méthode selon une quelconque des revendications précédentes dans laquelle les parties des plantes sont sélectionnées dans le groupe comprenant les cellules, les tissus, les organes, les racines, les tiges, les branches, les feuilles, et les mélanges de ceux-ci.

7. Plante laticifère ou une partie de celle-ci génétiquement modifiée pour contenir au moins une séquence nucléotidique codant pour au moins une molécule d'ARNi qui interfère avec l'activité polyphenoloxidase (PPO) spécifique du latex, résultant en un temps de polymérisation retardé ou une amélioration de la fluidité, respectivement, du latex sécrété par la dite plante ou partie de celle-ci comparé à la plante sauvage respective ou partie de celle-ci, dans laquelle le dit ARNi, au moins un, est dirigé spécifiquement contre (i) l'UTR 5' ou 3' du transcrit ARNm de la polyphenoloxidase spécific au latex, (ii) la séquence nucléotidique de l'ARNm qui code pour le domaine tyrosinase, et/ou (iii) la séquence nucléotidique de l'ARNm qui code pour le peptide de transition de la polyphenoloxidase spécifique au latex.

8. Plante laticifère ou une partie de celle-ci selon la revendication 7 dans laquelle la polyphenoloxidase spécifique du latex est représentée par la SEQ ID No. 2.

9. Plante laticifère ou une partie de celle-ci selon la revendication 7 ou 8 sélectionnée dans le groupe de familles de plantes comprenant les astéracées, les euphorbiacées et les moracées.

10. Plante laticifère ou une partie de celle-ci selon la revendication 9 sélectionnée dans le groupe comprenant Agoseris glauca, Apios americana, Apocynum androsaemifolium, Apocynum cannabinum, Asclepias asperula, Asclepias brachystephana, Asclepias californica, Asclepias decumbens, Asclepias eriocarpa, Asclepias erosa, Asclepias galioides, Asclepias hallii, Asclepias incarnata, Asclepias involucrata, Asclepias lanceolata, Asclepias latifolia, Asclepias mexicana, Asclepias valifolia, Asclepias pumila, Asclepias purpurascens, Asclepias quadrifolia, Asclepias rubra, Asclepias speciosa, Asclepias subulata, Asclepias sullivantii, Asclepias syriaca, Asclepias tuberosa, Asclepias viridiflora, Chrysothamnus graveolens, Chrysothamnus nauseosus, Chrysothamnus viscidiflorus, Cynanchum acutum, Eucomnia ulmoides, Euonymus europaeus, Euonymus hamiltonianus, Euonymus hamiltonianus maackii, Euonymus hamiltonianus sieboldianus, Euonymus japonicus, Euonymus latifolius, Euonymus verrucosus, Euphorbia lathyris, Hevea brasiliensis, Hymenoxys richardsonii, Jatropha spec., Lactuca sativa, Lactuca serriola, Marsdenia tenacissima, Nerium oleander, Parthenium argentatum, Scorzonera acanthoclada, Scorzonera albicaulis, Scorzonera divaricata, Scorzonera hissaricata, Scorzonera racemosa, Scorzonera tau-saghyz, Scorzonera tragapogonoides, Scorzonera turkestania, Scorzonera virgata, Solidago canadensis scabra, Solidago fistulosa, Solidago leavenworthii, Solidago rigida, Sonchus oleraceus, Sonchus asper, Taraxacum hybernum, Taraxacum koksaghyz, Taraxacum megalorrhizon, et Taraxacum officinale.

11. Plante laticifère ou une partie de celle-ci selon la revendication 9 ou 10 sélectionnée dans le groupe comprenant Hevea brasiliensis, Parthenium argentatum, Sonchus asper, Chrysothamnus nauseosus, Taraxacum koksaghyz, et Taraxacum officinale.

12. Plante laticifère ou une partie de celle-ci selon une quelconque des revendications 7 à 11 dans laquelle les parties des plantes sont sélectionnées dans le groupe comprenant les cellules, les tissus, les organes, les racines, les tiges, les branches, les feuilles, et les mélanges de ceux-ci.


### SEQUENCE LISTING

<110> Fraunhofer Gesellschaft
<120> Method for improving the crop of laticiferous plants
<130> 1086
<160> 18
<170> PatentIn version 3.3
<210> 1
   <211> 3624
   <212> DNA
   <213> Taraxacum officinale
<220>
   <221> 5'UTR
   <222> (1)..(1525)
<220>
   <221> misc_feature
   <222> (666)..(666)
   <223> n is a, g, c, or t
<220>
   <221> CDS
   <222> (1526)..(3325)
<220>
   <221> bipartite transit_peptide
   <222> (1526)..(1807)
<220>
   <221> misc_structure
   <222> (1526)..(1678)
   <223> envelope transfer peptide
<220>
   <221> misc_structure
   <222> (1679)..(1807)
   <223> stroma-lumen transfer peptide
<220>
   <221> misc_structure
   <222> (1808)..(2896)
   <223> tyrosinase domain
<220>
   <221> misc_structure
   <222> (2897)..(3325)
   <223> c-terminus
<220>
   <221> 3'UTR
   <222> (3326)..(3624)
<400> 1
   tggttgttcc ttgttaaatt agtaaatgat gatgttgata tattaaccta aaagaatcaa     60
   atgatcatgt gcccccaaaa tggagtatgt ttaggttatt aaccgaaaga agcaaatgat    120
   tatgttgata catttgattg attaaacgtt tcataattag taagttagat atattgtttc    180
   ataattagta agttagatat attgtcataa cctgcatttc ccaataaaat caatacattt    240
   aaactatgat cattaggaag tacatactat actatactcc ttcaagataa gcagatataa    300
   aaatccctaa atagctttgg aatttcacca tttattttct atataaatga aaggtagatg     360
   attttgggag ttatcaaatg atacatagtg cgatacagtc aaaaccttgt agacttggtg     420
   atggatgagc agggtaatag tactcttgag cttcggtttg gaacatattg tacgttgtgt     480
   tatcaagtcg cggtgttttg tgagggtgtc atatatggaa attgagagag attggaagtt     540
   gttttgtaat atgatagagt taaagaatgg tatcattatt ttgaaggtac tgagtgatat     600
   ttataatata gtaaagatat tataaaaagt attcttatac ataatgggtt taaataattg     660
   taaggnaagg aaataaatta atgtattaag aatttaaaaa gtgaaatggg aatggatgtc     720
   gagacttcgg gacccctgcc acacaactcc ttccactcgg agcccgagct agcccaggaa     780
   caatatgtcc gagccctccc tgaatcctga gtatagcccg aacccccctc tccataatat     840
   cattacactt agtcttatat taaacaaaca aaaaaatctt catatcggtt ttgtttaaat     900
   caataaaagc aacacaaaag gttgaatgaa ttgtcgaagt tatataatat atcaaataaa     960
   agttgtgaca ttttttaaat caaagtgtat taaggttgaa gtatcttaaa actcgaactt    1020
   acttgccaaa gttatataat ataatacata aacatgtaac tttttctaaa ttaaagtgta    1080
   ttaagcaggt tgaagtgtat taaagtgtat taagcaggtt gaagtgtatt aaagtgtatt    1140
   aaaagcaaca caaaaggttg aatgaattgt cgaagttata taatatatca aataaaagtt    1200
   gtgacatttt ttaaatcaaa gtgtattaag gttgaagtat cttaaaactc gaacttactt    1260
   gccaaagtta tacggagtat aatataatac ataaacatgt aactttttct aaattaaagt    1320
   gtattaagca ggttgaagta ttttaaaatt cgaacttata cgttaattac aaattaaagt    1380
   attttaatat aagtgataat tgatcactcg tttagaactt ttagtaatta taatacgata    1440
   aacatgggtt aaactttatt aggaagcttc tataaatata ttgctctcca ccgttatttt    1500
   aattacattc aaaccaagac taaat atg gca tcc ctt gca cca tct cca acc    1552
                               Met Ala Ser Leu Ala Pro Ser Pro Thr
                               1               5
   act acc ctc acc ccc act ggc gga agg agc ttc tct tcc tcc tcc aac    1600
   Thr Thr Leu Thr Pro Thr Gly Gly Arg Ser Phe Ser Ser Ser Ser Asn
   10                  15                  20                  25
   tac tct ttt tcc ttc tct ttt aat tca act caa gtt ccc aca cca aaa    1648
   Tyr Ser Phe Ser Phe Ser Phe Asn Ser Thr Gln Val Pro Thr Pro Lys
                   30                  35                  40
   acc ctc aac cat cgc cat gca gtg acg tca tgc aaa agt gca gat gac    1696
   Thr Leu Asn His Arg His Ala Val Thr Ser Cys Lys Ser Ala Asp Asp
               45                  50                  55
   cac cac cat gat ata gac tcg gga aca ctt gat aga aga aac gtc cta    1744
   His His His Asp Ile Asp Ser Gly Thr Leu Asp Arg Arg Asn Val Leu
           60                  65                  70
   tta cgt ctc gga ggt ctc tat ggt gcc gct gcc act ttt ggt tct aat    1792
   Leu Arg Leu Gly Gly Leu Tyr Gly Ala Ala Ala Thr Phe Gly Ser Asn
       75                  80                  85
   tca ctg gcc ttt gca gat ccg atc atg gca ccg gac ctc aca caa tgt    1840
   Ser Leu Ala Phe Ala Asp Pro Ile Met Ala Pro Asp Leu Thr Gln Cys
   90                  95                  100                 105
   ggt ccg gcc gac att cca gca ggt ggt ata gtc aca gac tgt tgc cct 1888
   Gly Pro Ala Asp Ile Pro Ala Gly Gly Ile Val Thr Asp Cys Cys Pro
   110 115 120
   cca ttc acc aca aag att caa gat ttc aaa ctt ccc ccg ccg tca aac 1936
   Pro Phe Thr Thr Lys Ile Gln Asp Phe Lys Leu Pro Pro Pro Ser Asn
   125 130 135
   acc ctc cgt atc cgg cca gcc gct cat ttg gtt gat aaa gat tac ata 1984
   Thr Leu Arg Ile Arg Pro Ala Ala His Leu Val Asp Lys Asp Tyr Ile
   140 145 150
   gac aaa ttc aat aaa gct atc ggg ctc atg aaa gct ctc cca gat gac 2032
   Asp Lys Phe Asn Lys Ala Ile Gly Leu Met Lys Ala Leu Pro Asp Asp
   155 160 165
   gat cca cgt agt ttc aag caa caa gct gtt gtt cat tgt gcc tat tgc 2080
   Asp Pro Arg Ser Phe Lys Gln Gln Ala Val Val His Cys Ala Tyr Cys
   170 175 180 185
   gat ggc gca tat gag caa gtc ggc tac cca ggt ctg gac ctt caa gtc 2128
   Asp Gly Ala Tyr Glu Gln Val Gly Tyr Pro Gly Leu Asp Leu Gln Val
   190 195 200
   cat aac tca tgg ctg ttc ttc cct ttc cat cgt tgt tac ttg tac ttt 2176
   His Asn Ser Trp Leu Phe Phe Pro Phe His Arg Cys Tyr Leu Tyr Phe
   205 210 215
   ttc gag aaa att tgt ggc aaa tta atc aat gac cca act ttc gca att 2224
   Phe Glu Lys Ile Cys Gly Lys Leu Ile Asn Asp Pro Thr Phe Ala Ile
   220 225 230
   ccg ttt tgg aac tgg gac gca gct gat ggg atg aca atc cct gac att 2272
   Pro Phe Trp Asn Trp Asp Ala Ala Asp Gly Met Thr Ile Pro Asp Ile
   235 240 245
   tac aca aat tcg aaa tct ccg tta tac gat act ctt cgc gat gca aaa 2320
   Tyr Thr Asn Ser Lys Ser Pro Leu Tyr Asp Thr Leu Arg Asp Ala Lys
   250 255 260 265
   cat caa cca cca tct gtg gtt gac ctt gac tac aat ggt gtt gac gaa 2368
   His Gln Pro Pro Ser Val Val Asp Leu Asp Tyr Asn Gly Val Asp Glu
   270 275 280
   aat ctt agt cct tcg gaa caa aca tct aca aat ctc aca att atg tat 2416
   Asn Leu Ser Pro Ser Glu Gln Thr Ser Thr Asn Leu Thr Ile Met Tyr
   285 290 295
   aga caa atg gtg tct agt gcc aaa act gct agt ctt ttc atg ggt agc 2464
   Arg Gln Met Val Ser Ser Ala Lys Thr Ala Ser Leu Phe Met Gly Ser
   300 305 310
   cct tat cgt gct ggc gat gag cct aac cct ggt ggt ggc acg tta gag 2512
   Pro Tyr Arg Ala Gly Asp Glu Pro Asn Pro Gly Gly Gly Thr Leu Glu
   315 320 325
   aac ata cca cac ggt ccg gta cat att tgg aca gga gat agg aac caa 2560
   Asn Ile Pro His Gly Pro Val His Ile Trp Thr Gly Asp Arg Asn Gln
   330 335 340 345
   cca aat ggt gaa aac atg ggt aac ttt tat tcg gct gga aaa gac cca 2608
   Pro Asn Gly Glu Asn Met Gly Asn Phe Tyr Ser Ala Gly Lys Asp Pro
   350 355 360
   att ttc tat gca cat cat gcg aat gtc gat aga atg tgg tca att tgg 2656
   Ile Phe Tyr Ala His His Ala Asn Val Asp Arg Met Trp Ser Ile Trp
   365 370 375
   aaa act cta ggt gga aga agg aaa gac ttt acc gat agc gac tgg ctt      2704
   Lys Thr Leu Gly Gly Arg Arg Lys Asp Phe Thr Asp Ser Asp Trp Leu
       380                 385                 390
   gat tct tcg ttc tcg ttc tat gat gag aac gcc gag ttt gtt cgg gtc      2752
   Asp Ser Ser Phe Ser Phe Tyr Asp Glu Asn Ala Glu Phe Val Arg Val
       395                 400                 405
   aag gtg agg gat tgt ctc gac tcc aag aag ctt ggt tat gct tat gaa      2800
   Lys Val Arg Asp Cys Leu Asp Ser Lys Lys Leu Gly Tyr Ala Tyr Glu
   410                 415                 420                 425
   gat gta aag ata cca tgg cta aaa acc aaa cct cgt tca aaa tac gga      2848
   Asp Val Lys Ile Pro Trp Leu Lys Thr Lys Pro Arg Ser Lys Tyr Gly
                   430                 435                 440
   aag tct agg aga aag gca gtg ggc cac aag cac gct gtt gcc cga gcc      2896
   Lys Ser Arg Arg Lys Ala Val Gly His Lys His Ala Val Ala Arg Ala
               445                 450                 455
   gat gaa ctc ata ccc ttt gca aaa gat gtt ttt ccg gcg agt ctt gat      2944
   Asp Glu Leu Ile Pro Phe Ala Lys Asp Val Phe Pro Ala Ser Leu Asp
           460                 465                 470
   aag gtc atg atc aag gtc ctg gtt cca agg ccg aag aag tcg agg agc      2992
   Lys Val Met Ile Lys Val Leu Val Pro Arg Pro Lys Lys Ser Arg Ser
       475                 480                 485
   aag aaa caa aaa gat gaa gaa gag gag gtt ttg gtt gta gaa gga att      3040
   Lys Lys Gln Lys Asp Glu Glu Glu Glu Val Leu Val Val Glu Gly Ile
   490                 495                 500                 505
   gaa gtg aag aga aac gag ttt gtg aag ttt gat gta ttt gtg gat gat      3088
   Glu Val Lys Arg Asn Glu Phe Val Lys Phe Asp Val Phe Val Asp Asp
                   510                 515                 520
   gat gat gac gag gtg agg gcc act gcg gat aag acg gaa ttc gcc gga      3136
   Asp Asp Asp Glu Val Arg Ala Thr Ala Asp Lys Thr Glu Phe Ala Gly
               525                 530                 535
   agt ttt gtg aat gtc cct cat gtg cat aag cat gag aag aat gtg acg      3184
   Ser Phe Val Asn Val Pro His Val His Lys His Glu Lys Asn Val Thr
           540                 545                 550
   acg cga ttg agg tta ggg ata agt gaa ctt ttg gac gat ttg gga gct      3232
   Thr Arg Leu Arg Leu Gly Ile Ser Glu Leu Leu Asp Asp Leu Gly Ala
       555                 560                 565
   gat gat gat gac aat gta ttg gtg aca ttg gtg ccc aaa acc gaa ggt      3280
   Asp Asp Asp Asp Asn Val Leu Val Thr Leu Val Pro Lys Thr Glu Gly
   570                 575                 580                 585
   ggt gaa gtt tcc att gga atg atc aaa atc gag tat gag gat tga          3325
   Gly Glu Val Ser Ile Gly Met Ile Lys Ile Glu Tyr Glu Asp
                   590                 595
   taaaaagaca tgcatactct ttttaatata aataaaataa aattaagtag atttcgagct    3385
   ttctgaaaag gtttcttgcc ttaggcgatt ctactcttca ttgaactcga caatgaaatt    3445
   ctacaatgca taaaacacta tagcgtgctc atgtaagata tatttataaa atctaagtta    3505
   tgacatgttt aataaataaa atgttttctc tgtactatat attgaaaatt cagtcgttat    3565
   tgaactaaga tatcaaaacg atattgatca gttgatccaa aaaaaaaaaa aaaaaaaaa     3624
<210> 2
   <211> 599
   <212> PRT
   <213> Taraxacum officinale
<400> 2
   Met Ala Ser Leu Ala Pro Ser Pro Thr Thr Thr Leu Thr Pro Thr Gly
   1               5                   10                  15
   Gly Arg Ser Phe Ser Ser Ser Ser Asn Tyr Ser Phe Ser Phe Ser Phe
               20                  25                  30
   Asn Ser Thr Gln Val Pro Thr Pro Lys Thr Leu Asn His Arg His Ala
           35                  40                  45
   Val Thr Ser Cys Lys Ser Ala Asp Asp His His His Asp Ile Asp Ser
       50                  55                  60
   Gly Thr Leu Asp Arg Arg Asn Val Leu Leu Arg Leu Gly Gly Leu Tyr
   65                  70                  75                  80
   Gly Ala Ala Ala Thr Phe Gly Ser Asn Ser Leu Ala Phe Ala Asp Pro
                   85                  90                  95
   Ile Met Ala Pro Asp Leu Thr Gln Cys Gly Pro Ala Asp Ile Pro Ala
               100                 105                 110
   Gly Gly Ile Val Thr Asp Cys Cys Pro Pro Phe Thr Thr Lys Ile Gln
           115                 120                 125
   Asp Phe Lys Leu Pro Pro Pro Ser Asn Thr Leu Arg Ile Arg Pro Ala
       130                 135                 140
   Ala His Leu Val Asp Lys Asp Tyr Ile Asp Lys Phe Asn Lys Ala Ile
   145                 150                 155                 160
   Gly Leu Met Lys Ala Leu Pro Asp Asp Asp Pro Arg Ser Phe Lys Gln
                   165                 170                 175
   Gln Ala Val Val His Cys Ala Tyr Cys Asp Gly Ala Tyr Glu Gln Val
               180                 185                 190
   Gly Tyr Pro Gly Leu Asp Leu Gln Val His Asn Ser Trp Leu Phe Phe
           195                 200                 205
   Pro Phe His Arg Cys Tyr Leu Tyr Phe Phe Glu Lys Ile Cys Gly Lys
       210                 215                 220
   Leu Ile Asn Asp Pro Thr Phe Ala Ile Pro Phe Trp Asn Trp Asp Ala
   225                 230                 235                 240
   Ala Asp Gly Met Thr Ile Pro Asp Ile Tyr Thr Asn Ser Lys Ser Pro
   245 250 255
   Leu Tyr Asp Thr Leu Arg Asp Ala Lys His Gln Pro Pro Ser Val Val
   260 265 270
   Asp Leu Asp Tyr Asn Gly Val Asp Glu Asn Leu Ser Pro Ser Glu Gln
   275 280 285
   Thr Ser Thr Asn Leu Thr Ile Met Tyr Arg Gln Met Val Ser Ser Ala
   290 295 300
   Lys Thr Ala Ser Leu Phe Met Gly Ser Pro Tyr Arg Ala Gly Asp Glu
   305 310 315 320
   Pro Asn Pro Gly Gly Gly Thr Leu Glu Asn Ile Pro His Gly Pro Val
   325 330 335
   His Ile Trp Thr Gly Asp Arg Asn Gln Pro Asn Gly Glu Asn Met Gly
   340 345 350
   Asn Phe Tyr Ser Ala Gly Lys Asp Pro Ile Phe Tyr Ala His His Ala
   355 360 365
   Asn Val Asp Arg Met Trp Ser Ile Trp Lys Thr Leu Gly Gly Arg Arg
   370 375 380
   Lys Asp Phe Thr Asp Ser Asp Trp Leu Asp Ser Ser Phe Ser Phe Tyr
   385 390 395 400
   Asp Glu Asn Ala Glu Phe Val Arg Val Lys Val Arg Asp Cys Leu Asp
   405 410 415
   Ser Lys Lys Leu Gly Tyr Ala Tyr Glu Asp Val Lys Ile Pro Trp Leu
   420 425 430
   Lys Thr Lys Pro Arg Ser Lys Tyr Gly Lys Ser Arg Arg Lys Ala Val
   435 440 445
   Gly His Lys His Ala Val Ala Arg Ala Asp Glu Leu Ile Pro Phe Ala
   450 455 460
   Lys Asp Val Phe Pro Ala Ser Leu Asp Lys Val Met Ile Lys Val Leu
   465 470 475 480
   Val Pro Arg Pro Lys Lys Ser Arg Ser Lys Lys Gln Lys Asp Glu Glu
   485 490 495
   Glu Glu Val Leu Val Val Glu Gly Ile Glu Val Lys Arg Asn Glu Phe
   500 505 510
   Val Lys Phe Asp Val Phe Val Asp Asp Asp Asp Asp Glu Val Arg Ala
           515                 520                 525
   Thr Ala Asp Lys Thr Glu Phe Ala Gly Ser Phe Val Asn Val Pro His
       530                 535                 540
   Val His Lys His Glu Lys Asn Val Thr Thr Arg Leu Arg Leu Gly Ile
   545                 550                 555                 560
   Ser Glu Leu Leu Asp Asp Leu Gly Ala Asp Asp Asp Asp Asn Val Leu
                   565                 570                 575
   Val Thr Leu Val Pro Lys Thr Glu Gly Gly Glu Val Ser Ile Gly Met
               580                 585                 590
   Ile Lys Ile Glu Tyr Glu Asp
           595
<210> 3
   <211> 22
   <212> DNA
   <213> Artificial
<220>
   <223> Primer LZ50
<400> 3
   caamcamcct mcgtgtccgg cc 22
<210> 4
   <211> 22
   <212> DNA
   <213> Artificial
<220>
   <223> Primer LZ45
<220>
   <221> misc_feature
   <222> (6)..(6)
   <223> n is inosine
<220>
   <221> misc_feature
   <222> (8)..(8)
   <223> n is inosine
<220>
   <221> misc_feature
   <222> (14)..(14)
   <223> n is inosine
<400> 4 tggtgnangg rggncaacaa tc 22
<210> 5
   <211> 22
   <212> DNA
   <213> Artificial
<220>
   <223> Primer LZ60
<400> 5
   gcaaagggta tgagttcatc gg 22
<210> 6
   <211> 22
   <212> DNA
   <213> Artificial
<220>
   <223> Primer LZ62
<400> 6
   aagatgtttt tccggcgagt ct 22
<210> 7
   <211> 24
   <212> DNA
   <213> Artificial
<220>0̸
   <223> Primer LZ55
<400> 7
   catcatgcga atgtcgatag aatg 24
<210> 8
   <211> 20
   <212> DNA
   <213> Artificial
<220>
   <223> Primer LZ56
<400> 8
   ccacctgctg gaatgtcggc 20
<210> 9
   <211> 31
   <212> DNA
   <213> Artificial
<220>
   <223> Primer LZ74
<400> 9
   aaactcgaga tatggcatcc cttgcaccat c 31
<210> 10
   <211> 24
   <212> DNA
   <213> Artificial
<220>
   <223> Primer LZ73
<400> 10
   caatcctcat actcgatttt gatc 24
<210> 11
   <211> 30
   <212> DNA
   <213> Artificial
<220>
   <223> Primer 5' race
<400> 11
   caggtgaaca atattcatca gtggagccac 30
<210> 12
   <211> 26
   <212> DNA
   <213> Artificial
<220>
   <223> Primer 3' race
<400> 12
   aaagatgttt ttccggcgag tcttga 26
<210> 13
   <211> 27
   <212> DNA
   <213> Artificial
<220>
   <223> Primer RNAi_for
<400> 13
   tttggtacca tccattggcc tttgcag 27
<210> 14
   <211> 30
   <212> DNA
   <213> Artificial
<220>
   <223> Primer RNAi_rev
<400> 14
   tttgcggccg cgtggtggaa gtttgaagtc 30
<210> 15
   <211> 10
   <212> PRT
   <213> Taraxacum officinale
<400> 15
Asp Glu Leu Ile Pro Phe Ala Lys Asp Val
   1 5 10
<210> 16
   <211> 10
   <212> PRT
   <213> Taraxacum officinale
<400> 16
Asp Pro Ile Met Ala Pro Asp Leu Thr Gln
   1 5 10
<210> 17
   <211> 8
   <212> PRT
   <213> Taraxacum officinale
<400> 17
Ala Leu Pro Asp Asp Asp Pro Arg
   1 5
<210> 18
   <211> 8
   <212> PRT
   <213> Taraxacum officinale
<400> 18
   Ser Pro Leu Tyr Asp Thr Leu Arg
   1 5